# EUROPEAN PATENT SPECIFICATION

(11) **EP 1 281 377 B1**
(45) Date of publication and mention of the grant of the patent: **12.09.2007**
(21) Application number: 02254837.4
(22) Date of filing: 10.07.2002
(51) Int. Cl.: A61F 9/00, A61F 9/007, A61M 1/00

(54) **Surgical flow restrictor and filter**
Chirurgischer Durchflussbegrenzer sowie Filter
Filtre et limiteur chirurgical de débit

(30) Priority: 03.08.2001 US 922473
(43) Date of publication of application: 05.02.2003
(73) Proprietor: Circuit Tree Medical, Inc., Mission Viejo, CA 92691 (US)
(72) Inventor: Urich, Alex, Mission Viejo, California 92692 (US)
(74) Representative: Wombwell, Francis

(56) References cited:
- EP-A- 0 998 978
- US-A- 3 812 855
- US-A- 4 061 143
- US-A- 4 393 879
- US-A- 4 813 926
- US-A- 5 324 254

## Description

### BACKGROUND OF THE INVENTION

### 2. Field of the Invention

The present application relates to a filter and flow restrictor for a medical aspiration system.

### 3. Background

The lens of a human eye may develop a cataracteous condition which affects a patients vision. Cataracteous lenses are sometimes removed and replaced in a procedure commonly referred to as phacoemulsification. Phaco procedures are typically performed with an ultrasonically driven handpiece which is used to break the lens. The broken lens is removed through an aspiration line that is coupled to the handpiece.

The handpiece has a tip which is inserted through an incision in the cornea. The handpiece typically contains a number of ultrasonic transducers that convert electrical power into a mechanical oscillating movement of the tip. The distal end of the tip has an opening that is in fluid communication with the aspiration line. The distal end of the tip also has a sleeve which has an opening in fluid communication with an irrigation line. The irrigation line is typically connected to a bottle that can provide irrigation fluid to the surgical site.

The oscillating movement of the tip will break the lens into small pieces. The lens pieces and irrigation fluid are drawn into the aspiration line through the opening of the tip. When performing a phaco procedure it is essential to maintain a positive pressure within the anterior chamber of the eye. A negative pressure may cause the cornea to collapse. To maintain a positive chamber pressure the system is configured to provide a flowrate through the irrigation tube that is greater than the flowrate through the aspiration tube.

It has been found that the aspiration tube may become occluded during a procedure. The occlusion will increase the vacuum pressure within the aspiration line. When the occlusion is cleared the anterior chamber may be instantaneous exposed to a high vacuum pressure. The vacuum pressure may cause the cornea to collapse.

Examples of prior art arrangements can be found in US-A-4 393 879 (Milgrom Hyman T) and US-A-4 813 926 (Kerwin Michael J).

### BRIEF SUMMARY OF THE INVENTION

A flow restrictor that includes a filter located within a filter housing and a flow restrictor coupled to the housing as set forth in the appended claims.

### BRIEF DESCRIPTION OF THE DRAWINGS

Figure 1 is an illustration of a medical system with a filter/flow restrictor;
Figure 2 is a cross-sectional view of an aspiration tube assembly of the system;
Figure 3 is a graph showing a flowrate versus a line vacuum pressure for, an aspiration system with a flow restrictor, and an aspiration system without a flow restrictor;
Figure 4 is a graph showing the line vacuum pressure versus the pressure within a cornea.

### DETAILED DESCRIPTION

Disclosed is a medical system that includes a filter/flow restrictor which limits the pressure drop within an aspiration system. The restrictor may include a flow restrictor that is coupled to a filter housing. The flow restrictor restricts the pressure drop within the aspiration system. The housing contains a filter which prevents occlusion of the flow restrictor. When used in an opthtalmic procedure the flow restrictor may limit the minimum pressure within a cornea and prevent corneal collapse.

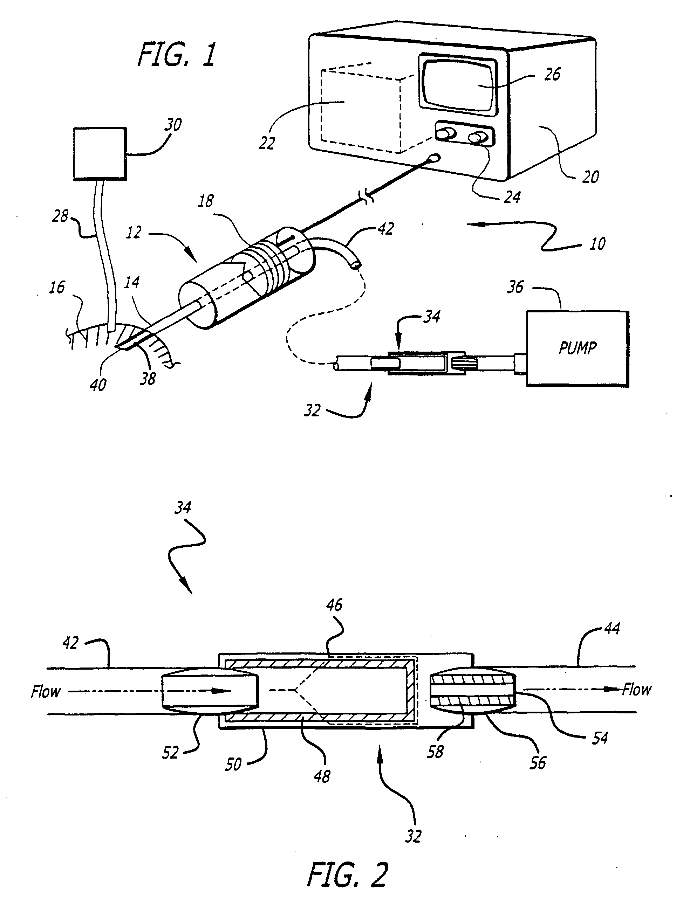

Referring to the drawings more particularly by reference numbers, Figure 1 shows an embodiment of a medical system 10. The system 10 may include an ultrasonically driven handpiece which has a tip 14 that can be inserted into a cornea 16. The tip 14 may also be referred to as a cutting element. The handpiece 12 may include one or more ultrasonic transducers 18 that convert electrical power into mechanical movement of the tip 14. The handpiece 12 is typically held by a surgeon who performs a surgical procedure with the system 10. By way of example, the system 10 can be used to perform a phacoemulsification procedure to break and aspirate a lens of the cornea 16.

The handpiece 12 may be connected to a console 20 of the system 10. The console 20 may contain a control circuit 22 that provides a driving signal to the transducers 18. The console 20 may have input knobs or buttons 24 that allow the surgeon to vary different parameters of the system 10. The console 20 may also have a readout display 26 that provides an indication of the power level, etc. of the system 10.

The system 10 may include an irrigation tube 28 that is connected to an irrigation bottle 30. The irrigation tube 28 can be inserted into the cornea 16. The irrigation bottle 30 may contain an irrigation fluid that flows into the cornea 16 through the irrigation tube 28.

The medical system 10 may further have an aspiration system 32 that aspirates the irrigation fluid and broken lens out of the cornea 16. The aspiration system 32 may include an aspiration tube assembly 34 that is connected to the handpiece 12 and a vacuum pump 36. The aspiration tube assembly 34 is in fluid communication with an inner channel 38 and an opening 40 of the tip 14. The vacuum pump 36 creates a negative pressure within the aspiration tube assembly 34 to induce a flow of irrigation fluid and emulsified tissue out of the cornea 16. The pump 36 is configured so that the flowrate through the irrigation tube 28 is slightly greater than the flowrate through the aspiration tube assembly 34.

As shown in Figure 2, the aspiration tube assembly 34 may include an input tube 42 and an output tube 44 that are coupled to a flow restrictor assembly 46. The flow restrictor assembly 46 includes a filter 48 that is located within a filter housing 50. The filter 48 may be a mesh type device that filters out particles and contaminates in the fluid flowing through the housing 50. It is preferable to have the filter 48 pressed into the housing 50 so that there is not a space between the outer filter surface and the inner housing surface. Press fitting the filter 48 into the filter housing 50 prevents air pockets from forming in the housing 50 and allows for proper pump priming of the restrictor 46.

The input tube 42 may include an input luer 52 that couples the tube 42 to the housing 50. The input luer 52 is preferably inserted into the filter 48 to prevent fluid from becoming trapped in the entrance of the housing 50. It is desirable to provide a filter 48 that has an outer diameter that is no greater than twice the diameter of the input luer 52. It has been found that such a ratio will insure that the restrictor 46 will be properly primed during aspiration. By way of example, the filter 48 may have a diameter of approximately 0.1 inch.

The restrictor assembly 46 includes a flow restrictor 54 coupled to the filter housing 50. The flow restrictor 54 limits the variance in flowrate of the irrigation fluid and the corresponding pressure drop across the aspiration system 32.

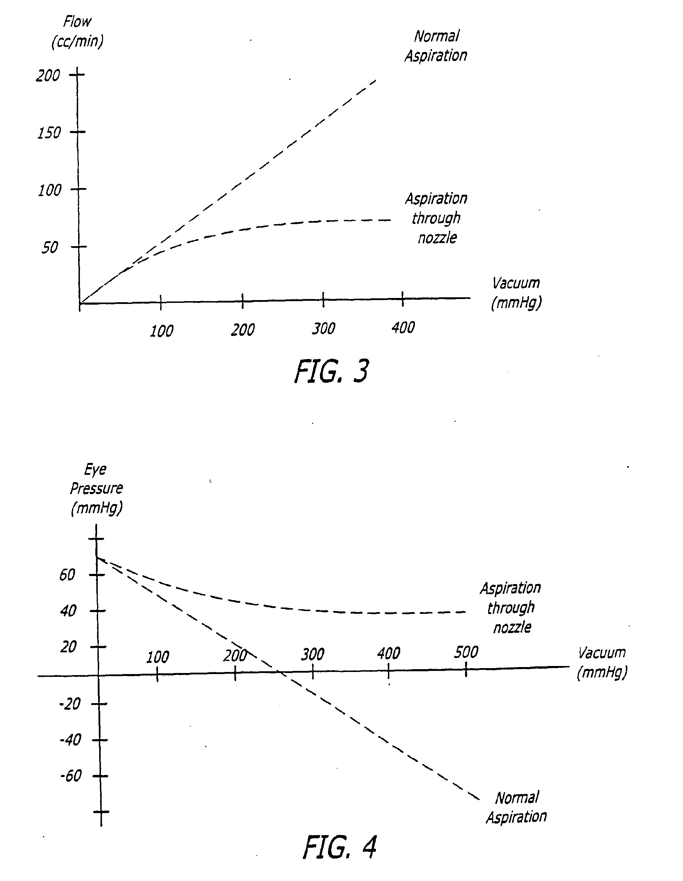

Figure 3 shows the vacuum pressure versus flowrate for an aspiration system with a flow restrictor, and a system without a restrictor. The vacuum pressure depicted is downstream from the restrictor. As can be seen a non-restrictor system will have a linear increase in flow with an increase in vacuum pressure. In contrast to the prior art, the flow restrictor 54 of the restrictor creates a non-linear increase in the flowrate that levels off after the vacuum pressure exceeds a certain level. The restriction of fluid flow limits the pressure drop in the cornea as shown in Figure 4 to prevent negative chamber pressure as indicated by the graph. By utilizing a flow restrictor, the aspiration system can prevent a negative pressure in the cornea anterior chamber that will cause a corneal collapse.

The flow restrictor 54 is integrated into an output luer 56 that is attached to the filter housing 50 and pressed into the output tube 44. The output luer 56 may have a detachable scaling insert 58 that defines the diameter of the flow restrictor 54. The diameter of the flow restrictor 54 may be varied by inserting a different scaling insert 58. The diameter of the flow restrictor 54 defines the upper limit of the flowrate in the aspiration system (see Fig. 3). The operator can vary the upper flowrate by inserting a different insert 58 and/or luer 56 into the tube assembly 34. By way of example, the flow restrictor 54 may have a diameter between 0.1 and 1.0 millimeters and a length at least 25.4 millimeters. This range will provide the nozzle effect shown in Figures 3 and 4 when fitted with convention asporation tubing found in medical systems. For example, conventional aspiration tubing diameters may range from 1.52 to 2.54 millimeters.

The filter 48 and housing 50 are preferably constructed from a disposable material. To insure that the restrictor 46 has capacity for an entire procedure it is preferable to provide a filter 48 which has a volume that can capture two cataract lenses. By way of example, the filter 48 may have a volume of approximately 1 cubic centimeter.

Referring to Figs. 1 and 2, in operation, an operator attaches a restrictor 46 to the input 42 and output 44 tubes. A medical procedure is then performed on a cornea 16. The aspiration system 32 may develop an occlusion which increases the downstream vacuum pressure of the system. The flow restrictor 54 limits the flowrate from the cornea 16 to prevent corneal collapse when the occlusion is cleared from the system. The filter 48 prevents the flow restrictor 54 from being occluded to allow for normal fluid flow during a procedure. After the procedure is completed the flow restrictor 46 is replaced with another restrictor unit.

While certain exemplary embodiments have been described and shown in the accompanying drawings, it is to be understood that such embodiments are merely illustrative of and not restrictive on the broad invention as defined in the claims and that this invention not be limited to the specific constructions and arrangements shown and described, since various other modifications may occur to those ordinarily skilled in the art.

## Claims

1. A flow restrictor assembly (46) for a medical aspiration system (32), comprising:
a filter housing (50);
a non-adjustable flow restrictor (54) coupled to said filter housing; and
a filter (48) located within said filter housing,
**characterised in that** the non-adjustable flow restrictor (54) is located within an output luer (56) attached to said filter housing.

2. The flow restrictor of claim 1, wherein said flow restrictor (54) has a diameter between 0.1 to 1 millimeters.

3. The flow restrictor of claim 1, wherein said output luer (56) includes a scaling insert (58),

4. An aspiration tube assembly (32) for a medical system comprising:
an input tube (42);
an input luer(52) coupled to said input tube, said input luer having a diameter;
a filter housing (50) coupled to said input luer;
a filter(48) located within said filter housing,and
a non-adjustable flow restrictor (54) coupled to said filter housing,
**characterised in that** said non-adjustable flow restrictor (54) is located within an output luer (56) attached to said filter housing.

5. The aspiration tube assembly of claim 4, wherein said input luer (52) is pressed into said filter (48).

6. The aspiration tube assembly of claim 4, wherein said filter (48) is pressed into said filter housing (50).

7. The aspiration tube assembly of claim 4, wherein said flow restrictor (54) has a diameter between 0.1 to 1 millimeters.

8. The aspiration tube assembly of claim 4, wherein said output luer (56) includes a scaling insert (58).

9. The aspiration tube assembly of claim 4, wherein said filter (48) has a diameter that is no greater than twice the diameter of said input luer (52).

## Patentansprüche

1. Durchflussbegrenzeranordnung (46) für ein medizinisches Absaugsystem (32), mit:
einem Filtergehäuse (50);
einem nicht verstellbaren, an das Filtergehäuse gekoppelten Durchflussbegrenzer (54); und
einem in dem Filtergehäuse angeordneten Filter (48),
**dadurch gekennzeichnet, dass**
der nicht verstellbare Durchflussbegrenzer (54) innerhalb eines an dem Filtergehäuse angebrachten Ausgangs-Luer-Verbinder (56) angebracht ist.

2. Durchflussbegrenzer nach Anspruch 1,
wobei der Durchflussbegrenzer (54) einen Durchmesser von 0,1 bis 1 Millimeter aufweist.

3. Durchflussbegrenzer nach Anspruch 1,
wobei der Ausgangs-Luer-Verbinder (56) einen Skaliereinsatz (58) umfasst.

4. Absaugröhrenanordnung (32) für ein medizinisches System, mit einer Eingangsröhre (42);
einem an die Eingangsröhre gekoppelten Eingangs-Luer-Verbinder (52), wobei der Eingangs-Luer-Verbinder einen Durchmesser aufweist;
einem an den Eingangs-Luer-Verbinder gekoppelten Filtergehäuse (50), und
einem nicht verstellbaren, an das Filtergehäuse gekoppelten Durchflussbegrenzer (54),
**dadurch gekennzeichnet, dass**
der nicht verstellbare Durchflussbegrenzer (54) in einem an das Filtergehäuse angebrachten Ausgangs-Luer-Verbinder (56) angeordnet ist.

5. Absaugröhrenanordnung nach Anspruch 4,
wobei der Eingangs-Luer-Verbinder (52) in den Filter (48) gedrückt ist.

6. Absaugröhrenanordnung nach Anspruch 4,
wobei der Filter (48) in das Filtergehäuse (50) gedrückt ist.

7. Absaugröhrenanordnung nach Anspruch 4,
wobei der Durchflussbegrenzer (54) einen Durchmesser von 0,1 bis 1 Millimeter aufweist.

8. Absaugröhrenanordnung nach Anspruch 4,
wobei der Ausgangs-Luer-Verbinder (56) einen Skaliereinsatz (58) umfasst.

9. Absaugröhrenanordnung nach Anspruch 4,
wobei der Filter (48) einen Durchmesser aufweist, der nicht größer ist als zweimal der Durchmesser des Eingangs-Luer-Verbinder (52).

## Revendications

1. Ensemble de limiteur de débit (46) pour un système d'aspiration médical (32), comprenant :
- un logement de filtre (50) ;
- un limiteur de débit non réglable (54) couplé audit logement de filtre ; et
- un filtre (48) situé dans ledit logement de filtre,
**caractérisé en ce que** le limiteur de débit non réglable (54) est situé dans un luer de sortie (56) fixé audit logement de filtre.

2. Limiteur de débit selon la revendication 1, dans lequel ledit limiteur de débit (54) a un diamètre compris entre 0,1 et 1 millimètre.

3. Limiteur de débit selon la revendication 1, dans lequel ledit luer de sortie (56) comprend un insert à gradin (58).

4. Ensemble de tube d'aspiration (32) pour un système médical comprenant:
- un tube d'entrée (42)
- un luer d'entrée (52) raccordé audit tube d'entrée, ledit luer d'entrée ayant
- un certain diamètre ;
- un logement de filtre (50) raccordé audit luer d'entrée ;
- un filtre (48) situé dans ledit logement de filtre, et
- un limiteur de débit non réglable (54) couplé audit logement de filtre,
**caractérisé en ce que** ledit limiteur de débit non réglable (54) est situé dans un luer de sortie (56) fixé audit logement de filtre.

5. Ensemble de tube d'aspiration selon la revendication 4, dans lequel ledit luer d'entrée (52) est introduit par pression dans ledit filtre (48).

6. Ensemble de tube d'aspiration selon la revendication 4, dans lequel ledit filtre (48) est introduit par pression dans ledit logement de filtre (50).

7. Ensemble de tube d'aspiration selon la revendication 4, dans lequel ledit limiteur de débit (54) a un diamètre compris entre 0,1 et 1 millimètre.

8. Ensemble de tube d'aspiration selon la revendication 4, dans lequel ledit luer de sortie (56) comprend un insert à gradin (58).

9. Ensemble de tube d'aspiration selon la revendication 4, dans lequel ledit filtre (48) a un diamètre qui n'est pas supérieur à deux fois le diamètre dudit luer d'entrée (52).
